# EUROPÄISCHE PATENTSCHRIFT

(11) **EP 0 888 746 B1**
(45) Veröffentlichungstag und Bekanntmachung des Hinweises auf die Patenterteilung: **17.10.2007**
(21) Anmeldenummer: 98108131.8
(22) Anmeldetag: 05.05.1998
(51) Int. Cl.: A61B 17/28

(54) **Chirurgisches Rohrschaftinstrument**
Surgical instrument having a tubular shaft
Instrument chirurgical à tige tubulaire

(30) Priorität: 02.07.1997 DE 19728114
(43) Veröffentlichungstag der Anmeldung: 07.01.1999
(73) Patentinhaber: Aesculap AG & Co. KG, 78532 Tuttlingen (DE)
(72) Erfinder: Mayenberger, Rupert, 78239 Rielasingen (DE); Morales, Pedro, 78532 Tuttingen (DE); Weisshaupt, Dieter, 78194 Immendingen (DE)
(74) Vertreter: Boehme, Ulrich

(56) Entgegenhaltungen:
- EP-A- 0 573 817
- WO-A-96/11636
- US-A- 5 275 613
- US-A- 5 275 615
- US-A- 5 509 923

## Beschreibung

Die Erfindung betrifft ein chirurgisches Rohrschaftinstrument mit einem rohrförmigen Schaft und einem daran anschließenden Griffteil, mit einem um eine quer zur Längsachse des Schaftes verlaufende Drehachse verschwenkbaren Werkzeug und mit einem vom Griffteil aus in Längsrichtung des Schaftes verschiebbaren Schubelement im Schaft, welches mit dem Werkzeug über von der Drehachse des Werkzeuges entfernt angeordnete Vor- und Rücksprünge derart in Eingriff steht, daß das Werkzeug beim Verschieben des Schubelementes um seine Drehachse verschwenkbar ist.

Derartige chirurgische Rohrschaftinstrumente sind beispielsweise aus der US-Patentschrift 5 509 923 bekannt. Zum Verschwenken der beiden Teile des Werkzeuges tragen diese ein Zahnsegment, das mit einem länglichen Zahnsegment am Schubelement kämmt und somit die Translationsbewegung des Schubelementes in eine Rotationsbewegung der Werkzeugteile übersetzt.

Rohrschaftinstrumente dieser Art weisen oft einen sehr geringen Durchmesser auf, so daß sich Schwierigkeiten ergeben, bei den kleinen Durchmessern die notwendige Stabilität zu erreichen. Daher gelingt es häufig nicht, mit derartigen Rohrschaftinstrumenten die Schließkräfte für das Werkzeug zu erzeugen, die wünschenswert wären.

Insbesondere besteht bei der bekannten Konstruktion von Rohrschaftinstrumenten die Gefahr, daß die Wirkverbindung zwischen den Vorsprüngen und den Rücksprüngen durch Verformung des eingesetzten Materials aufgehoben wird, die miteinander kämmenden Vor- und Rücksprünge können "springen" oder vollständig außer Eingriff geraten.

Es ist Aufgabe der Erfindung, ein gattungsgemäßes Rohrschaftinstrument so auszugestalten, daß trotz kleiner Abmessungen zuverlässig große Drehmomente auf die Werkzeuge übertragen werden können.

Diese Aufgabe wird bei einem Rohrschaftinstrument der eingangs beschriebenen Art erfindungsgemäß dadurch gelöst, daß am Schubelement ein ringförmiger Käfig angeordnet ist, der die Drehachse des Werkzeuges sowie die Eingriffsstelle des Vorsprungs und des Rücksprungs umgibt und dessen Innenwand den Vorsprung oder Rücksprung des Schubelementes trägt.

Die Verwendung eines ringförmigen Käfigs, also eines in sich geschlossenen Bauteils, ergibt eine wesentlich erhöhte Stabilität der wirksamen Flächen des Schubelementes, und damit wird sichergestellt, daß eine Verformung dieser die Schubbewegung in eine Rotationsbewegung umsetzenden Teile auch bei hoher Belastung nicht auftritt. Die Vor- und Rücksprünge befinden sich im Inneren des Käfigs, ein Ausweichen der entsprechenden Teile nach außen ist durch die Käfigbildung ausgeschlossen.

Grundsätzlich kann das Werkzeug ein einziges verschwenkbares Teil umfassen, bei einer besonders bevorzugten Ausführungsform ist aber vorgesehen, daß das Werkzeug zwei gegensinnig und koaxial verschwenkbare Teile umfaßt, die beide über Vor- oder Rücksprünge im Innern des Käfigs des Schubelementes mit diesem in Wirkverbindung stehen.

Dabei ist es freigestellt, ob der Vorsprung am Käfig oder am Werkzeug angeordnet ist, wesentlich ist lediglich, daß ein Vorsprung und ein Rücksprung miteinander in Wirkverbindung stehen.

Bei einer bevorzugten Ausführungsform können beispielsweise Vor- und Rücksprünge durch Zahnsegmente gebildet werden, es ist aber genauso gut möglich, jeweils nur einen einzigen in einen Rücksprung eingreifenden Vorsprung vorzusehen.

Bei gegensinnig verschwenkbaren Werkzeugteilen ist es vorteilhaft, wenn der Käfig zwei seitlich nebeneinanderliegende Längsarme umfaßt, von denen jeder einen Vor- oder Rücksprung für eines der beiden Teile trägt, daß die beiden Teile nebeneinander um die Drehachse schwenkbar gelagert sind und daß je ein Teil und ein Längsarm in einer Ebene liegen und miteinander in Wirkverbindung stehen.

Auf diese Weise ist eine besonders platzsparende Anordnung möglich, es können nämlich die Teile des Werkzeuges nebeneinander liegen, die Längsarme des Schubelementes befinden sich dann jeweils in der Ebene des ihnen zugeordneten Werkzeugteils, während das jeweils andere Werkzeugteil an den Längsarmen vorbei verschwenkt werden kann.

Günstig ist es dabei auch, wenn die Längsarme an ihrem freien Ende durch eine quer zu ihrer Längsrichtung verlaufende Brücke miteinander verbunden sind, diese Brükke schließt den von den Längsarmen umgebenen Innenraum ab und bildet somit den in sich geschlossenen Käfig aus.

In sich geschlossen bedeutet dabei nicht, daß der Käfig allseits geschlossen ist, sondern nur daß der Käfig ringförmig geschlossen ist, seitlich kann der Käfig ohne weiteres offen sein. Ringförmig ist dabei allgemein zu verstehen, die Form des Innenraums des Käfigs kann verschieden sein, beispielsweise ist die Form eines Langloches günstig.

Bei einer bevorzugten Ausführungsform wird der Käfig zwischen zwei Lagerarmen des Schaftes längsverschieblich geführt, die eine Lagerwelle für das Werkzeug zwischen sich halten. Diese Lagerarme dienen somit gleichzeitig als Verdrehsicherung für den Käfig und das mit dem Käfig verbundene Schubelement.

Die nachfolgende Beschreibung bevorzugter Ausführungsformen der Erfindung dient im Zusammenhang mit der Zeichnung der näheren Erläuterung. Es zeigt:
- Figur 1:: eine schematische Seitenansicht eines Rohrschaftinstrumentes mit einem verschwenkbaren Werkzeug;
- Figur 2:: eine vergrößerte Detailansicht des Werkzeugbereichs im Längsschnitt mit zwei gegeneinander verschwenkbaren Werkzeugteilen in Offenstellung;
- Figur 3:: eine Ansicht ähnlich Figur 2 in Schließstellung;
- Figur 4:: eine Schnittansicht längs Linie 4-4 in Figur 3;
- Figur 5:: eine perspektivische Ansicht des die Lagerstelle der Werkzeugteile umgebenden Käfigs bei dem Ausführungsbeispiel der Figuren 1 bis 4;
- Figur 6:: eine Ansicht ähnlich Figur 3 bei einem Rohrschaftinstrument, bei dem Vorsprung und Rücksprung an Käfig und Werkzeugteilen gegenüber dem Ausführungsbeispiel der Figuren 1 bis 5 vertauscht sind und
- Figur 7:: eine Schnittansicht längs Linie 7-7 in Figur 6.

Das in der Zeichnung dargestellte Rohrschaftinstrument 1 umfaßt einen länglichen, rohrförmigen Schaft 2 und ein Griffteil 3, welches eine mit dem Schaft 2 fest verbundene Branche 4 und eine gegenüber dem Schaft 2 verschwenkbar gelagerte Branche 5 umfaßt. Die Branche 5 ist gelenkig mit einem stangenförmigen Schubelement 6 im Inneren des Schaftes 2 verbunden, welches beim Verschwenken der Branche 5 gegenüber der feststehenden Branche 4 im Schaft 2 längsverschieblich ist.

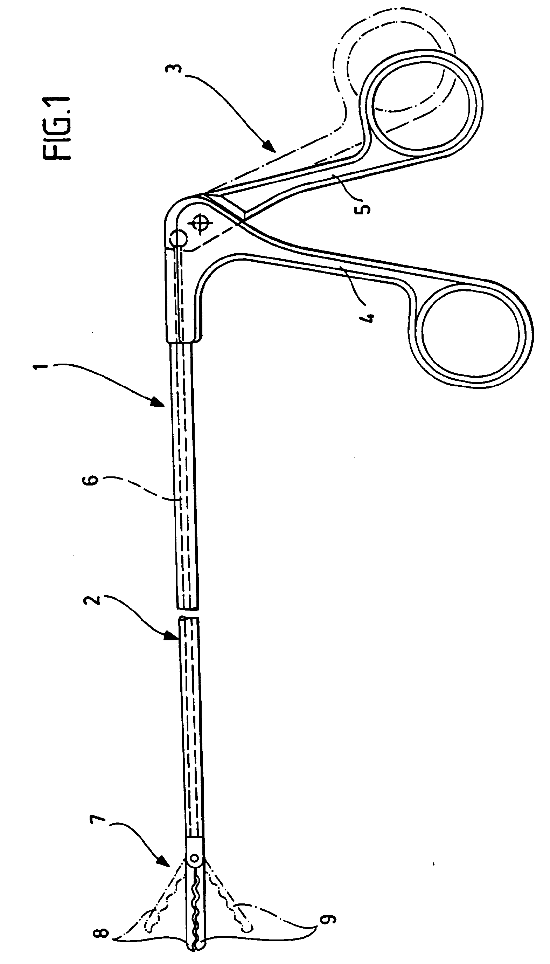

Am freien Ende des Schaftes 2 ist um eine quer zur Längsrichtung des Schaftes 2 verlaufende Drehachse verschwenkbar ein Werkzeug 7 gelagert, welches im dargestellten Ausführungsbeispiel zwei in entgegengesetzter Richtung verschwenkbare Werkzeugteile 8, 9 umfaßt. Diese werden mittels des Schubelementes 6 zwischen einer Schließstellung (in Figur 1 in ausgezogenen Linien) und einer Öffnungsstellung (in Figur 1 in strichpunktierten Linien) verschwenkt, wobei die Translation des Schubelementes durch geeignete Getriebemittel in eine Rotationsbewegung der Werkzeugteile 8, 9 übersetzt wird.

In das freie Ende des Schaftes 2 ist ein Lagerstück 10 eingeschoben, welches den Schaft 2 am freien Ende verschließt. Dieses Lagerstück 10 weist zwei parallel nebeneinander in Längsrichtung des Schaftes 2 verlaufende Lagerarme 11, 12 auf, die zwischen sich einen Abstand einhalten und somit einen nach oben, nach unten und nach vorn hin offenen Aufnahmeraum 13 ausbilden, der mit dem Innenraum des Schaftes 2 über einen zentralen Kanal 14 im Lagerstück 10 in Verbindung steht.

Zwischen den beiden Lagerarmen 11, 12 ist eine den Aufnahmeraum 13 quer durchsetzende Lagerwelle 15 gehalten, auf welcher nebeneinanderliegend die zwei Werkzeugteile 8, 9 drehbar gelagert sind. Die Lagerwelle 15 definiert somit die Drehachse der Werkzeugteile 8, 9.

Die Breite der Werkzeugteile 8, 9 ist halb so groß wie die Breite des Aufnahmeraumes 13, so daß die Werkzeugteile 8, 9 dadurch auch in Richtung der Lagerwelle 15 eine definierte Position einnehmen.

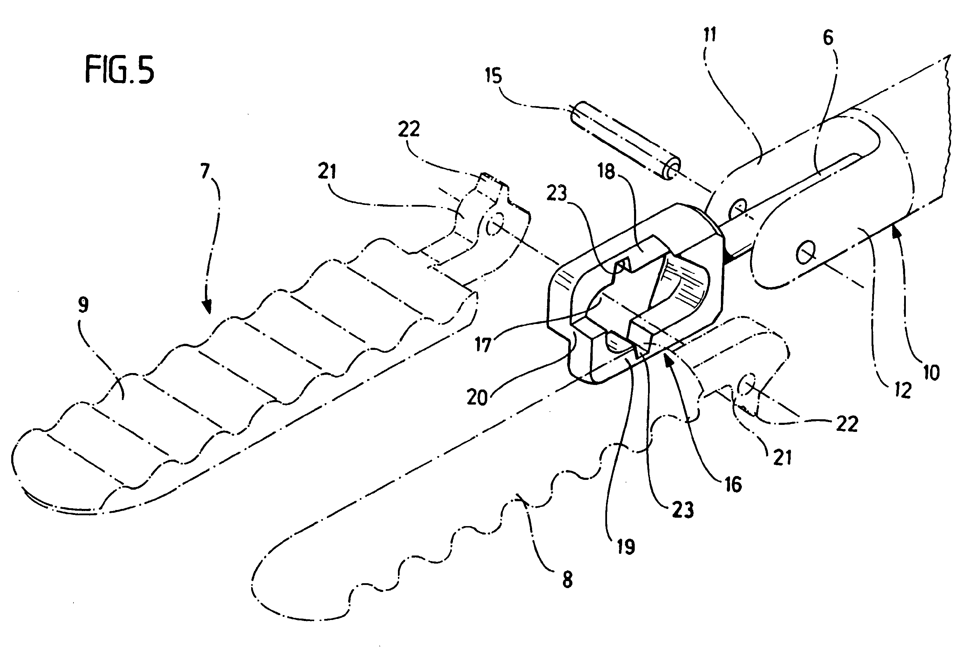

Das stangenförmige Schubelement 6 tritt durch den zentralen Kanal 14 in den Aufnahmeraum 13 ein und ist dort einstückig verbunden mit einem im wesentlichen quaderförmigen Käfig 16, dessen Breite der Breite des Aufnahmeraumes 13 entspricht. Dieser Käfig 16 (Figur 5) weist eine sich im wesentlichen über seine gesamte Länge erstreckende langlochförmige Ausnehmung 17 auf, so daß ein oberer Längsarm 18 und ein unterer Längsarm 19 ausgebildet werden, die an ihren freien Enden miteinander verbunden sind.

Die Breite der Längsarme 18 und 19 ist dabei nur halb so groß wie die Breite des Käfigs 16 insgesamt, und die Längsarme 18 und 19 sind seitlich gegeneinander versetzt, so daß die Verbindung der beiden Längsarme 18 und 19 am freien Ende durch eine quer verlaufende Brükke 20 erfolgt.

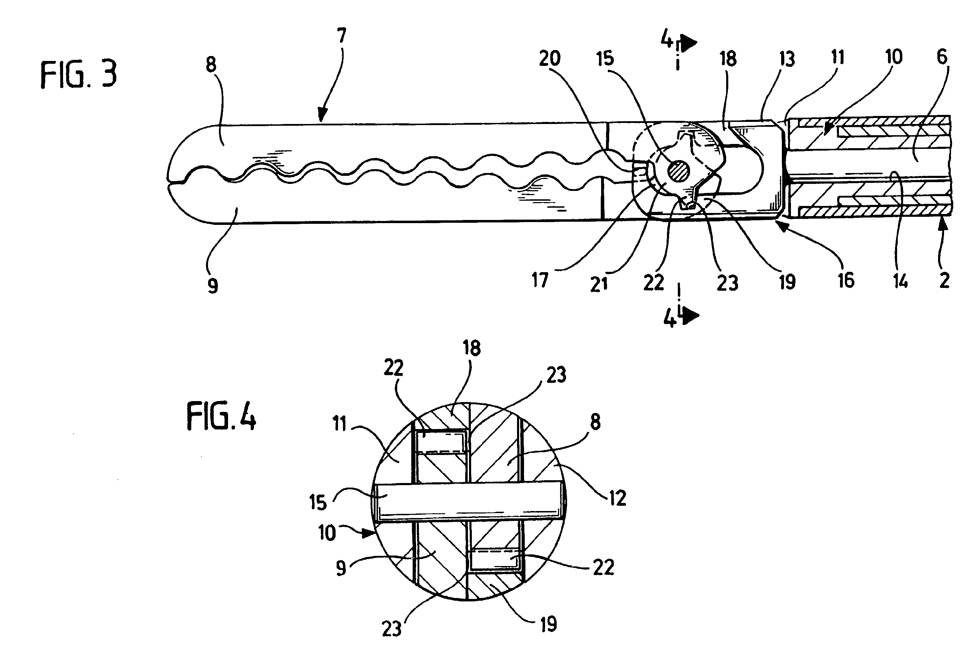

Der Käfig 16 umgibt die Lagerwelle 15, der obere Längsarm 18 ist oberhalb des Werkzeugteiles 9 angeordnet, der untere Längsarm 19 unterhalb des Werkzeugteiles 8 (Figur 4).

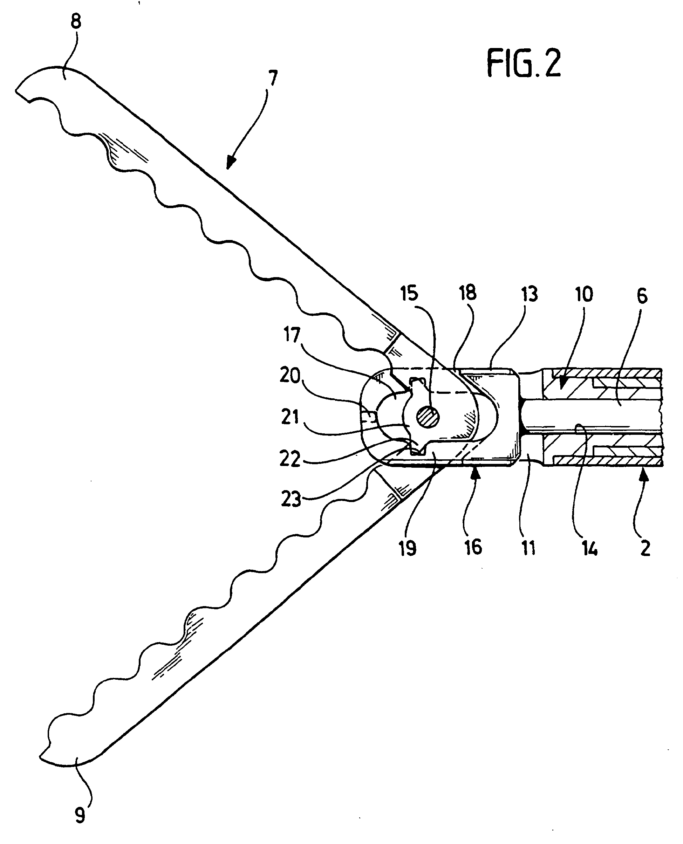

Die Werkzeugteile 7 und 8 umgeben die Lagerwelle 15 in Form eines Ringes 21, der beim Ausführungsbeispiel der Figuren 1 bis 5 einen radial abstehenden Vorsprung 22 trägt. Dieser taucht in einen Rücksprung 23 des in der Ebene des jeweiligen Werkzeugteiles liegenden Längsarmes des Käfigs 16 ein, wobei dieses Eintauchen nach Art des Eingriffs von Zähnen eines Zahnradgetriebes ausgebildet ist. Bei einer Längsverschiebung des Käfigs 16 ergibt sich dadurch eine Mitnahme des Ringes 21 des jeweiligen Werkzeugteils und damit eine Verschwenkung des Werkzeugteils um die durch die Lagerwelle 5 gebildete Drehachse. Die Verschwenkung der Werkzeugteile erfolgt dabei in entgegengesetzter Richtung, beim Vorschub des Käfigs 16 erfolgt beispielsweise bei dem dargestellten Ausführungsbeispiel eine Öffnung der beiden Werkzeugteile, beim Zurückschieben des Käfigs 16 dagegen ein Schließen.

Der Käfig 16 ist zwischen den beiden Lagerarmen 11, 12 unverdrehbar längsgeführt und füllt zusammen mit den Werkzeugteilen 8, 9 den Aufnahmeraum 13 zwischen den Lagerarmen 11, 12 aus.

Durch die Ausbildung des Käfigs 16 als geschlossener Körper wird eine Verformung der Längsarme 18, 19 auch dann ausgeschlossen, wenn über die Wirkverbindung zwischen Vorsprung 22 und Rücksprung 23 hohe Momente übertragen werden, der Eingriff des Vorsprunges 22 in den Rücksprung 23 bleibt also auch bei hoher Belastung erhalten.

Bei dem Ausführungsbeispiel der Figuren 1 bis 5 tragen die Werkzeugteile 8, 9 jeweils einen Vorsprung 22, der in einen Rücksprung 23 des Käfigs 16 eingreift.

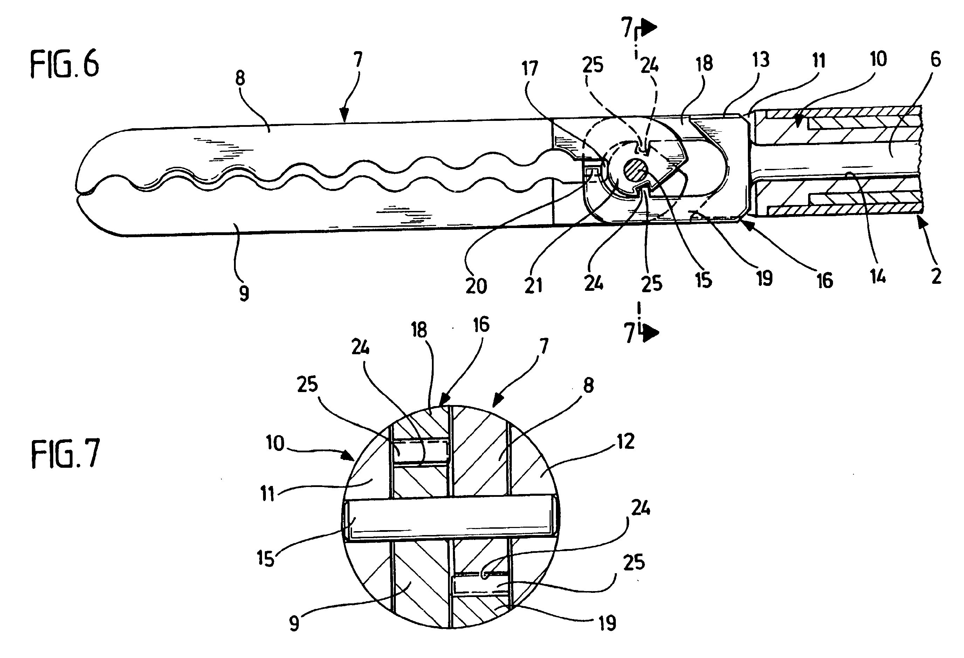

Bei dem Ausführungsbeispiel der Figuren 6 und 7, das sonst gleich aufgebaut ist und bei dem gleiche Teile daher dieselben Bezugszeichen tragen, ist diese Anordnung umgekehrt getroffen worden. Die Werkzeugteile 8, 9 weisen in diesem Falle Rücksprünge 24 auf, in die Vorsprünge 25 am Käfig 16 eingreifen.

Bei den dargestellten Ausführungsbeispielen erfolgt der Wirkeingriff zwischen Werkzeugteilen einerseits und Käfig andererseits jeweils nur durch einen Vorsprung und einen Rücksprung. Statt dessen wäre es ohne weiteres möglich, auch Zahnsegmente miteinander kämmen zu lassen, dies ist insbesondere dann von Vorteil, wenn grössere Schwenkbewegungen der Werkzeugteile gewünscht werden.

## Patentansprüche

1. Chirurgisches Rohrschaftinstrument mit einem rohrförmigen Schaft und einem daran anschließenden Griffteil, mit einem um eine quer zur Längsachse des Schaftes verlaufende Drehachse verschwenkbaren Werkzeug und mit einem vom Griffteil aus in Längsrichtung des Schaftes verschiebbaren Schubelement im Schaft, welches mit dem Werkzeug über von der Drehachse des Werkzeugs entfernt angeordnete Vor- und Rücksprünge derart in Eingriff steht, daß das Werkzeug beim Verschieben des Schubelementes um seine Drehachse verschwenkbar ist, **dadurch gekennzeichnet, daß** am Schubelement (6) ein ringförmiger Käfig (16) angeordnet ist, der die Drehachse des Werkzeuges (7) sowie die Eingriffstelle des Vorsprunges (22; 25) und des Rücksprunges (23; 24) umgibt und dessen Innenwand den Vorsprung (22) oder Rücksprung (24) des Schubelementes (6) trägt.

2. Instrument nach Anspruch 1, **dadurch gekennzeichnet, daß** das Werkzeug (7) zwei gegensinnig und koaxial verschwenkbare Teile (8, 9) umfaßt, die beide über Vor- oder Rücksprünge (22, 23; 24, 25) im Innern des Käfigs (16) des Schubelements (6) mit diesem in Wirkverbindung stehen.

3. Instrument nach Anspruch 2, **dadurch gekennzeichnet, daß** der Käfig (16) zwei seitlich nebeneinander liegende Längsarme (18, 19) umfaßt, von denen jeder einen Vor- oder Rücksprung (23, 25) für eines der beiden Teile (8, 9) trägt, daß die beiden Teile (8, 9) nebeneinander um die Drehachse schwenkbar gelagert sind und daß je ein Teil (8, 9) und ein Längsarm (19, 18) in einer Ebene liegen und miteinander in Wirkverbindung stehen.

4. Instrument nach Anspruch 3, **dadurch gekennzeichnet, daß** die Längsarme (18, 19) an ihrem freien Ende durch eine quer zu ihrer Längsrichtung verlaufende Brücke (20) miteinander verbunden sind.

5. Instrument nach einem der voranstehenden Ansprüche, **dadurch gekennzeichnet, daß** der Käfig (16) zwischen zwei Lagerarmen (11, 12) des Schaftes (2) längsverschieblich geführt ist, die eine Lagerwelle (15) für das Werkzeug (7) zwischen sich halten.

6. Instrument nach einem der voranstehenden Ansprüche, **dadurch gekennzeichnet, daß** die miteinander in Wirkverbindung stehenden Vor- und Rücksprünge durch Zahnsegmente gebildet werden.

## Claims

1. A surgical tubular-shafted instrument having a tubular shaft and a handle part connected thereto, having a tool pivotable about an axis of rotation which runs transversely to the longitudinal axis of the shaft and having a push element located in the shaft and displaceable from the handle part in the longitudinal direction of the shaft, which push element engages the tool, via projections and recesses arranged at a distance from the axis of rotation of the tool, in such a manner that the tool is pivotable about its axis of rotation upon displacement of the push element, **characterised in that** an annular cage (16) is arranged on the push element (6), surrounds the axis of rotation of the tool (7) as well as the engagement site of the projection (22; 25) and the recess (23; 24) and bears the projection (22) or recess (24) of the push element (6) at its inside wall.

2. An instrument according to claim 1, **characterised in that** the tool (7) comprises two parts (8, 9) pivotable coaxially and in opposite directions to one another, which parts (8, 9) are both in an operative connection with the push element (6) via projections or recesses (22, 23; 24, 25) within the cage (16) of the push element (6).

3. An instrument according to claim 2, **characterised in that** the cage (16) comprises two longitudinal arms (18, 19) lying laterally side by side, each arm bearing a projection or recess (23, 25) for one of the two parts (8, 9), **in that** the two parts (8, 9) are mounted side by side so as to be pivotable about the axis of rotation, and **in that** a part (8, 9) and a respective longitudinal arm (19, 18) lie in one plane and are in an operative connection with one another.

4. An instrument according to claim 3, **characterised in that** the longitudinal arms (18, 19) are connected to one another at their free end by a bridge (20) running transversely to their longitudinal direction.

5. An instrument according to any one of the preceding claims, **characterised in that** the cage (16) is guided between two bearing arms (11, 12) of the shaft (2) in a longitudinally displaceable manner, a bearing shaft (15) for the tool (7) being held between these bearing arms.

6. An instrument according to any one of the preceding claims, **characterised in that** the projections and recesses in an operative connection with one another are formed by toothed segments.

## Revendications

1. Instrument chirurgical à fût tubulaire, comportant un fût en forme de tube et une partie de préhension se raccordant à celui-ci, comportant un outil pivotant autour d'un axe de rotation s'étendant perpendiculairement à l'axe longitudinal du fût et comportant un élément de poussée dans le fût, déplaçable depuis la partie de préhension en direction longitudinale du fût, lequel est en engagement avec l'outil par l'intermédiaire de saillies et retraits agencés à distance de l'axe de rotation de l'outil, de telle sorte que l'outil peut être pivoté autour de son axe de rotation lors du déplacement de l'élément de poussée, **caractérisé en ce que** sur l'élément de poussée (6) est agencée une cage (16) annulaire qui entoure l'axe de rotation de l'outil (7) ainsi que le point d'engagement de la saillie (22 ; 25) et du retrait (23 ; 24) et dont la paroi intérieure porte la saille (22) ou le retrait (24) de l'élément de poussée (6).

2. Instrument selon la revendication 1, **caractérisé en ce que** l'outil (7) comprend deux parties (8, 9) pivotables en sens inverse et coaxialement, qui sont toutes les deux en liaison active avec l'élément de poussée (6) via des saillies ou retraits (22, 23 ; 24, 25) à l'intérieur de la cage (16) de l'élément de poussée (6).

3. Instrument selon la revendication 2, **caractérisé en ce que** la cage (16) comprend deux bras longitudinaux (18, 19) situés latéralement l'un à côté de l'autre, dont chacun porte une saillie ou un retrait (23, 25) pour une des deux parties (8, 9), **en ce que** les deux parties (8, 9) sont montées l'une à côté de l'autre de manière à pivoter autour de l'axe de rotation, et **en ce qu'**une partie respective (8, 9) et un bras longitudinal (19, 18) sont situés dans un plan et en liaison active l'un avec l'autre.

4. Instrument selon la revendication 3, **caractérisé en ce que** les bras longitudinaux (18, 19) sont reliés à leur extrémité libre par un pont (20) s'étendant transversalement à leur direction longitudinale.

5. Instrument selon l'une des revendications précédentes, **caractérisé en ce que** la cage (16) est guidée à déplacement longitudinal entre deux bras de montage (11, 12) du fût (2), lesquels maintiennent entre eux un arbre de montage (15) pour l'outil (7).

6. Instrument selon l'une des revendications précédentes, **caractérisé en ce que** les saillies et retraits en liaison active les uns avec les autres sont formés par des segments dentés.
